# EUROPEAN PATENT APPLICATION

(11) **EP 2 939 643 A1**
(43) Date of publication of application: **04.11.2015**
(21) Application number: 14166216.3
(22) Date of filing: 28.04.2014
(51) Int. Cl.: A61F 13/84

(54) **QUALITATIVE METHOD FOR DETERMINING IF A SURFACE HAS WICKING PROPERTIES**

(71) Applicant: The Procter & Gamble Company, Cincinnati, OH 45202 (US)
(72) Inventor: Su, Qi, 65824 Schwalbach (DE); Liput, Marta, 65824 Schwalbach (DE)
(74) Representative: Briatore, Andrea

(57) **Abstract**

The present invention relates to a method for determining if a surface of an object has wicking properties in a given time range. The method includes the steps of providing a surface to be tested and contacting it with a liquid while observing for the given time range if liquid is transferred to the surface in a direction having at least a component which is opposite to the gravitational force.

## Description

### FIELD OF THE INVENTION

Determining surface properties is a common interest in several industries. In particular in the industry of absorbent materials it can be necessary to determine if a surface of a given material has wicking properties.

Measurements of capillarity, permeability, capillary force and other parameters which are related to the wicking properties of a surface are known in the science and industry, however the measurement of these parameters involves the use of relatively complex methods which require skilled technicians, sophisticated lab equipment and a significant amount of time.

In some cases it may be desirable to have available a quick method which allows to qualitatively determine if a surface has wicking properties.

Wicking is defined as the capacity of a surface to draw fluids by capillary action.

Many fibrous materials used in the paper and absorbent articles industry must have good wicking surface properties, moreover many treatments (thermal, chemical, coating etc.) which are applied industrially to paper or nonwoven materials in order to change their properties or their appearance may affect wicking properties significantly.

Therefore there is a need for a quick and easy method which allows to verify qualitatively if a surface has inherently wicking properties, or if such surface has maintained its wicking properties after a treatment. In some cases there is also a need for a quick method to show convincingly that a surface has wicking properties.

### SUMMARY OF THE INVENTION

The present invention relates to a method for determining if a surface of an object has wicking properties in a given time range. The method includes the following steps:
a. Defining a time range *t* in seconds during which to observe wicking
a. Providing an object having a surface and positioning the surface so that, in a system of orthogonal coordinates x, y, z, oriented so that gravitational force acts along the z axis toward its negative direction, the surface to be tested faces the negative z axis,
b. Providing a liquid material having a liquid material surface facing the object surface
c. Move the surface of the object and the surface of the liquid relative to each other until they get in contact forming an interface and then immediately stop the motion.
d. After the relative motion has been stopped provide observation means to observe visually for the given time range *t* if some of said liquid keeps getting transferred through said interface to said object surface along a direction which (projected onto said system of orthogonal coordinates) has a at least component directed toward the positive z axis, i.e. in a direction opposite of the gravitational field.

### DETAILED DESCRIPTION OF THE INVENTION

Wicking is defined as the capacity of a material to draw fluids by capillary action. In the present invention the term wicking referred to a "surface" indicates that the materials from which the surface is made has wicking properties. Typically this will be the case when the surface is made of a fibrous material and if at least a part of the fibers forming the surface have wicking properties. Wicking is time dependent, in the sense that it typically requires some seconds for the wicking action to start effectively, therefore in this method we will associate the wicking properties to a time range of observation. In other words the method of the invention will be used to determine if a surface has wicking properties after a certain time of contact, for example if a certain surface has wicking properties at 2 seconds.

The term **"absorbent article"** is used herein in a broad sense including any article able to receive and/or absorb and/or contain and/or retain body fluids/bodily exudates such as menses, vaginal secretions, urine and faeces. Exemplary absorbent articles in the context of the present invention are disposable hygiene absorbent articles such as feminine hygiene absorbent articles, including tampons, adult incontinence pads and diapers for baby and adults. The term "disposable" is used herein to describe articles, which are not intended to be laundered or otherwise restored or reused as an article (i.e. they are intended to be discarded after a single use and preferably to be recycled, composted or otherwise disposed of in an environmentally compatible manner). Typical absorbent articles according to the present invention are sanitary napkins, panty liners, vaginal tampons, absorbent pads for low, moderate or high incontinence, baby diapers or pants, as well as diapers and pants for adult incontinence.

Absorbent articles according to the present invention include a surface which can be tested for wicking properties. The surface is not necessarily flat because the method can be performed also on a very small portion of surface.

Typically absorbent articles (with the exception of vaginal tampons) comprise a fluid pervious topsheet, a backsheet, which may be fluid impervious and/or may be water vapour and/or gas pervious, and an absorbent core comprised there between.

The invention is particularly suitable for determining if the surface of an absorbent article or of a material intended for use in an absorbent article has wicking properties in a given time range defined as *"t". "t"* is typically measured in seconds, and it is preferably in a range of from 1 to 60 seconds. More preferably is in a range of from 1 to 5 seconds, and for example is 2 seconds. To note the method provides information only on the specific portion of surface which is tested, so in case the properties of a surface vary from point to point, the method may need to be repeated in a representative set of locations in order to "map" the wicking properties of the whole surface.

In the case of an absorbent article the whole article can be used for testing, typically the surface to be tested will be the body facing surface of the absorbent article, where the topsheet is located. Alternatively the topsheet could be removed so to expose the surface of one of the internal layers. In this case the surface of the exposed internal layer can be tested.

In case it is desired to test the wicking properties in a range of *t* seconds of the surface of a component material for an absorbent article, a piece of that material can be used for this testing. The size of the piece of material can be any size which can be adapted to the measuring equipment and the dimension of the liquid surface.

The surface of the object to be tested is positioned (considering a system of orthogonal coordinates xyz wherein the gravitational force is exerted toward the negative z axis) so to face at least partially the negative z axis, in other words it is positioned at least partially facing down so that an object in contact with the surface and not attached to it would be pushed by the gravity to move accelerating toward the negative z direction.

The surface to be tested can have any shape because the test can be performed on a very small area with a single drop of liquid, but in general the surface to be tested will be flat or, preferably, slightly convex toward the negative z axis. Preferably, in order to obtain a better visibility, the surface to be tested is facing essentially down so that the z axis is essentially perpendicular to the surface in the point of contact with the liquid (as customary in geometry, in case the surface is convex in that point, the perpendicular direction to that surface is the direction of the line perpendicular to the tangent in that point to said curved surface)

A liquid material having a surface is also provided. Liquids to be used include any liquid but typically water can be used, water may contain salt in order to simulate body fluids or colorants to enhance visibility so that the liquid has a contrasting color with respect to the surface to be tested. The use of colored liquids is preferred as it makes it easier to verify if the liquid has been wicked.

Also artificial menstrual fluid (AMF as known in the art) could be used for this purposes.

Preferably the liquid surface is the curved surface of a liquid as it is pushed out of a thin tube having a diameter of less than 5mm or less than 3mm or less than 1mm such as a plastic tube, or a glass tube or a dropper or a pipette or a syringe. The liquid is typically connected to a mean for pushing the liquid. Due to its surface tension the liquid pushed out of this tube will form a curved surface, in general smaller tubes will provide curved surfaces with a smaller radius. A smaller radius allows a better and easier visualization of the wicking during the test. In this case before starting the test the liquid in the thin tube is slowly and slightly pushed out of said tube so to form this curved surface without letting the drop of liquid fall.

The liquid surface and the surface to be tested are carefully put in contact with each other.

Typically the surface to be tested is kept in a fixed position and the liquid surface at the top of said tube is moved by hand or via a transferring mean until it touches the surface to be tested. Typically the movement is operated in a direction essentially perpendicular to the surface to be tested. The preferred setup is that where the surface faces down and its perpendicular goes along the z axis and the liquid surface is moved along said z axis.

Once the surface to be tested and the liquid surface initiate the contact forming an interface the relative movement of the two surfaces is stopped. At this point the behaviour of the liquid is observed for "*t*" seconds. If the surface has wicking capacity it will be able to transfer some of the liquid from the liquid surface into the surface to be tested, this liquid moving against the force of gravity. This thanks to a capillary force which overcomes the action of gravity.

Even if a small amount of fluid is transferred the surface is considered to have wicking properties in the given timeframe *t*. A surface which does not have wicking properties will be wet only at the point of contact of the interface, while the fluid will not transfer beyond that point and will not move against the force of gravity.

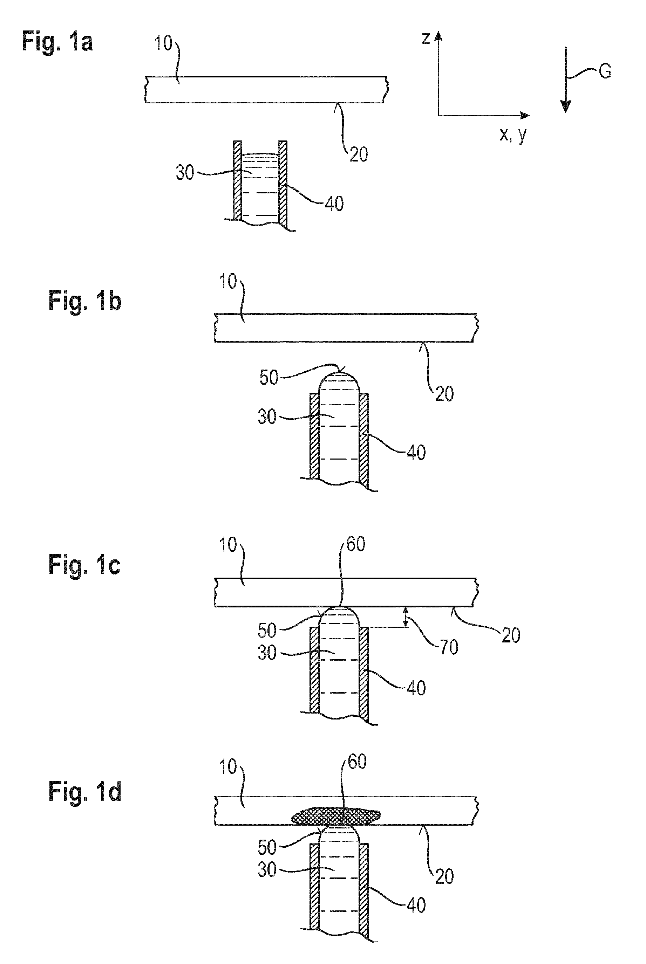

Fig. 1 represents a typical sequence for the method of the present invention. Fig. 1 a) represents the initial condition wherein an object 10 is placed in a fixed position facing down (toward the negative z axis, in a system where also the gravity force G is directed toward the negative z axis). The object 10 has a surface 20 to be tested for wicking properties. A colored liquid 30 is placed in a thin tube 40 such as the tip of a glass pipette (or of a syringe). In Fig. 1 b) the liquid 30 is slowly pushed in the pipette until its surface 50 forms a convex surface. In case the liquid does not form a convex surface when pushed out of the pipette, a pipette with a smaller diameter should be used.

In Fig. 1 c) it is shown how the pipette with the liquid forming a convex surface 50 is slowly moved toward the surface to be tested 20 until the surfaces 20 and 50 touch each other forming an interface 60. That the glass of the pipette does not enter in contact with the surface to be tested 20 so that a gap 70 between the pipette and the surface 20 is maintained.

Maintaining a fixed gap 70, the liquid is observed for "*t*" seconds. If the surface has wicking properties at least some of the fluid will transfer to the surface 20 through the interface 60 and this will be visible as the colored fluid will have colored in part the object 10. Fig. 1 d) shows the final situation where it is observed how a part of the fluid has moved from the pipette to the object 10 in a direction opposed to the force of gravity. This confirms that the surface 20 has wicking capacity.

## Claims

1. A method for determining if a surface of an object has wicking properties in a given time range said method including the following steps:
a. defining a time range *t* in seconds during which to observe wicking
a. providing an object having a surface and positioning said surface so that, in a system of orthogonal coordinates x/y/z, oriented so that gravitational force acts along the z axis toward its negative direction, said surface faces the negative z axis,
b. providing a liquid material having a liquid material surface facing said object surface
c. move said surface of said object and said surface of said liquid relative to each other until they get in contact forming an interface and then immediately stop the motion.
d. after said relative motion has been stopped provide observation means to observe visually for *t* seconds if some of said liquid keeps getting transferred through said interface to said object surface along a direction which (projected onto said system of orthogonal coordinates) has at least a component directed toward the positive z axis, in a direction opposite of said gravitational field.

2. A method according to claim 1 wherein *t* is comprised between 1 and 60 seconds.

3. A method according to claim 2 wherein *t* is comprised between 1 and 5 seconds.

4. A method according to any preceding claim wherein said liquid is in a liquid dispensing means

5. A method according to claim 4 wherein said liquid dispensing means comprise a tube and means for pushing liquid out of said tube.

6. A method according to claims 4-5 wherein said liquid dispensing means is able to form drops of said liquid.

7. A method according to claims 4-6 wherein said liquid dispensing means is a pipette or a dropper.

8. A method according to claims 4-7 wherein said liquid material surface is the convex surface of a drop of liquid still connected to said liquid dispensing means.

9. A method according to any preceding claim wherein said gravitational force is essentially perpendicular to said object surface in the point of contact with said liquid.

10. A method according to any preceding claim wherein said object surface facing the negative z axis is flat or convex.

11. A method according to any preceding claim wherein said observation means are a selected from a camera, a video camera or the eyes of an observer.

12. A method according to any preceding claim wherein said liquid has a contrasting color with respect to said surface.

13. A method according to any preceding claim wherein said object surface is the surface of an absorbent article.

14. A method according to claim 1-12 wherein said object surface is the surface of a component material for an absorbent article.
